# EUROPEAN PATENT APPLICATION

(11) **EP 4 649 927 A1**
(43) Date of publication of application: **19.11.2025**
(21) Application number: 24176680.7
(22) Date of filing: 17.05.2024
(51) Int. Cl.: A61F 13/15, A41B 9/00, A61F 13/49, A61F 13/505

(54) **WASHABLE ABSORBING UNDERGARMENT**

(71) Applicant: Essity Hygiene and Health Aktiebolag, 405 03 Göteborg (SE)
(72) Inventor: KNÖS, Anna, 405 03 Göteborg (SE); BLOMSTRÖM, Philip, 405 03 Göteborg (SE)
(74) Representative: Nederlandsch Octrooibureau

(57) **Abstract**

A washable absorbent undergarment (1), comprises a body fabric (2) forming a front side, a rear side and a crotch region and defining a waist opening and leg openings. An absorbent gusset (10) is located at an inside of the garment and comprises a garment-facing moisture barrier layer (12). The body fabric comprises a single layer of double-knit material extending to the leg openings.

## Description

### Field

The present disclosure relates to washable absorbent undergarments comprising an absorbent gusset, in particular, those intended for sanitary use in absorbing body fluids such as urine and vaginal fluids. The disclosure relates in particular to improvements that make such garments more discreet and pleasant to wear and to simplified and cost-effective methods of manufacture.

### Background

A washable absorbent undergarment may be worn for the purpose of absorbing body fluids such as urine and vaginal fluids. Such garments form an environmental advantageous alternative to disposable sanitary napkins and disposable absorbent undergarments. A washable absorbent undergarment typically comprises fabric panels of woven or knitted materials. Conventionally such a washable absorbent undergarment will also comprise an integral absorbent assembly or gusset located in the crotch region of the wearer when the undergarment is worn. After use the undergarment is washed or laundered before reuse. An example of such a washable absorbent undergarment is found in WO2023169665A1, in which a side edging is used to retain the absorbent assembly along the edges of the leg openings.

Depending upon the intended use, the absorbent gusset may vary in size and construction. It may extend over a smaller or larger area of the garment, e.g. depending on whether it is intended for day-time or night-time use. It may also be thicker or thinner, with additional wicking, distribution, barrier and absorbing layers. Nevertheless, in addition to being functional from an absorbing perspective, the resulting undergarment must inspire confidence and be comfortable to wear for prolonged periods as well as providing a good fit and being aesthetically pleasing.

A problem that may be encountered with such washable absorbent undergarments is their relative bulk. Not only do the multiple layers of the gusset increase the thickness of the undergarment but any seams and side edging to retain the gusset can be visible through outer clothing layers. This visible panty line is especially prominent where it coincides with the edges of the leg openings. It would be desirable to provide a washable absorbent undergarment that was less visible through outer clothing layers.

### Summary

This object may be at least partially achieved with an undergarment in accordance with claim 1 and a method of its manufacture according to claim 13.

The disclosed washable absorbent undergarment comprises a body fabric forming a front side, a rear side and a crotch region and defining a waist opening and leg openings with an absorbent gusset at an inside of the garment comprising a garment-facing moisture barrier layer. The body fabric comprises a single layer of double-knit material that extends to the leg openings.

As used herein, the term "absorbent undergarment" refers to garments that are worn and intended to be placed against the skin of the wearer with the intended function of absorbing and containing body fluids such as urine and vaginal fluids including menstrual fluid, without significant visible external trace. The undergarment may for example be underwear, underpants, a panty, or swimwear of fabric. The undergarment is intended for teens and adult users. The undergarment may be designed for male or female use. It will be understood that most conventional garments will have a limited degree of absorbency but that for conventional undergarments the containment of body fluids is not intended, and an unexpected leakage of any significant volume would be perceptible externally either as a damp patch or discoloration that may penetrate through an outer garment.

The absorbent undergarment according to the present disclosure is washable, i.e. intended to be laundered or otherwise restored after use for repeated reuse as a sanitary article. By "washable" it is meant that the absorbent undergarment may be cleaned by laundering. The absorbent undergarment may thus be subjected to an aqueous solution containing detergent without losing its structural features. This aqueous solution may be heated as part of the laundering process, e.g. to 30°C or 60°C. The term is also intended to mean that the absorbent undergarment can be washed and reused more than once. Washable may be understood as being suitable to undergo at least 10, such as at least 50, washing cycles at a temperature of at least 30°C, without disintegrating. During the washing, the absorbent gusset releases the absorbed fluids, thereby regenerating the absorptive functioning, enabling the absorbent gusset to be reused for the same purpose again. A disposable absorbent article is not constructed to be washable and reusable and is thus for single use only.

The body fabric according to the present disclosure comprises double-knit material, which in the present context is intended to denote a fabric created by interlocking two layers of fabric during the knitting process. The knitting process involves using two sets of needles that work together to create this double layer of fabric. The layers are knit together, resulting in a fabric that is not only thicker, but also more durable than other types of knit fabrics and offers good stretch. Due to its double-layer construction, double knit fabric is less likely to tear or wear out compared to other knit fabrics. It retains its shape well and is resistant to wrinkles. The body fabric may be an interlock fabric. Interlock knit fabric is a variation of rib knit. It has two rows of stitches created one behind the other, using two rows of needles that cross over each other and the rows become "interlocked" as the fabric is knitted. Because of these two rows, interlock knit fabric is known as a double-knit fabric. The interlocked, double rows create a fabric that is recognized by the rows or ribs of a 'V' shape down the face and back of the fabric. It gives the fabric a smooth consistent appearance on both sides, making it reversible. The fabric prevents runs and does not unravel or curl at the edges. In the context of the present disclosure, interlock knit fabric is used to denote a double-knit fabric in which the front and rear face are identical in all but optionally colour, whereas in the case of a more general double-knit fabric, the two layers may be distinct e.g. in terms of knit or yarn.

The gusset may be spaced from the leg openings by at least 2 mm, preferably at least 5 mm. This denotes the minimum spacing between an edge of the gusset and a corresponding edge of a leg opening and may be the case in the crotch region. In this manner, the change in thickness of the garment corresponding with the edge of the gusset will never coincide with the panty line corresponding to the edge of the leg opening.

The gusset may have any suitable size and shape according to the intended use. Its relative extent with respect to the garment will also depend on the style of the garment. The gusset may extend from the crotch to a maximum height of the leg opening at the front side and/or the rear side. The maximum height of the leg opening is defined by the closest distance to the waist opening. The gusset may extend from the crotch to the front and/or back side and may terminate below the maximum height of the leg opening e.g. at 90% or 80% or 70% of the distance to this point from the midpoint.

The gusset may be rectangular. In this context, "rectangular" is intended to denote that the gusset has an outer contour that is a four-sided shape, having two pairs of sides that are generally parallel to one another. The rectangular assembly or its components can be easily cut multiple times from a main material source (often a large sheet or roll of fabric or foil material), with a single cutting line forming two edges of adjacent components. Thus, the use of rectangular shaped gussets reduces the amount of cutting and minimizes waste. Furthermore, the cutting of rectangular contours from a main material source allows for multiple components to be cut directly adjacent to one another, without having to account for a positioning direction of each individual component in the manufacturing line. Thus, no material is wasted during production to account for variation in direction of each component.

At least 75% of the total length of the edges of the at least one rectangular shaped gusset may be straight edges. Thus at least 75% of the outer contour of the rectangular shaped gusset conforms to the rectangular shape, thereby limiting the amount of material wasted, while allowing for some minimal shape variations in the layer that are required to assure wearer comfort and prevent the article from bulging during use.

The width of the gusset may also be chosen according to the use and style of the garment. It may extend over the full width of the front side and/or the back side up to the above indicated minimum distance from the leg openings. It may also be narrower than the garment, wherein there is a spacing between the gusset and the leg openings on each side of the gusset. The spacing may taper from a minimum distance in the crotch region to a maximum spacing at the front or rear edge of the gusset. The maximum spacing on each side may be less than 100 mm or less than 30 mm or less than 20 mm at the front side and/or the rear side. The side edges of the gusset may be straight and may diverge from each other from the crotch region. They may diverge to a lesser extent than the respective portion of the front side and/or rear side between the leg openings so that the spacing between the gusset and the leg opening increases.

The gusset construction may be generally conventional for washable absorbent undergarments. It may comprise a body-facing top layerthat may be co-extensive with the moisture barrier layer. It will be understood that for the avoidance of ridges, exact coincidence of the edges of the different layers may be avoided. The top layer may itself be moisture absorbent or may serve primarily to allow fluids to penetrate to optional layers beneath. Such layers may include intermediate wicking, acquisition and/or absorbent layers between the top layer and the moisture barrier layer. These layers may form an absorbent core. The absorbent core may have a smaller extent than the top layer and/or the barrier layer.

The moisture barrier layer may be a woven or fibrous layer or a polymeric film. It may be hermetic or breathable, i.e. air and vapour permeable. It may comprise a thermoplastic film, such as polyurethane. The moisture barrier layer may comprise or consist of a weave or knit of activated carbon. Fibers used in the weave or knit may be of filament, stretch-broken or staple type and may be present in any filamentary form, including fibres and yarns that may contain activated carbon filaments of any length. Such activated carbon weave or knit may be commercially produced by the activation of the filaments within an existing fabric, either chemically or thermally.

As indicated above, the size of the gusset may depend on the intended use but also on its absorbency per unit area. A gusset with a thicker, more absorbent core may be smaller in area than a gusset with a thinner core but having the same overall capacity to absorb fluids. A wicking layer may be used to distribute fluid from a point of delivery throughout the gusset, allowing use of a thinner gusset. The gusset may cover an area of at least 10 cm² of the garment, such as between 10 cm² and 400 cm², or between 50 cm² and 100 cm².

In general, the gusset will be permanently attached to the body fabric. It is however not excluded that separable gussets may be provided, which may be washed separately or replaced. The gusset may be attached to the body fabric over its complete area or it may only be connected at individual points. The gusset and body fabric may be connected together along edges of the gusset. Some or all of the edges may be connected, e.g. only the side edges or only the front and rear edges.

Various methods may be used to attach the gusset to the body fabric. The gusset may be attached to the body fabric by an adhesive connection between the barrier layer and the body fabric. The barrier layer may be provided on its garment-facing surface with a pressure sensitive adhesive or a heat activated adhesive at the desired bonding positions. The adhesive may be applied by spray coating, laminating or any other appropriate procedure. Alternatively, a bonding member may be used, which may be a double-sided tape applied to the garment-facing side of the barrier layer or a single-sided tape applied onto the top sheet side of the gusset. Single-sided tape may stick to the inside of the garment or may wrap around at least in the crotch region to engage the top layer of the gusset and an outer side of the body fabric. The tape may be elastic e.g. in order to allow it to stretch together with the body fabric. The body fabric may, instead or in addition to bonding members, extend and wrap around the first and second side edges of the gusset in the crotch region and bond to the top layer of the gusset. Alternative connections may include stitching, welding, heat-sealing and combinations of the above. In particular, stitching may be applied at corners of the gusset in combination with adhesive connection.

According to an important aspect of the present disclosure, by the use of double-knit material, the garment may be embodied without seams at edges of the body fabric. In this context, seamless means that there is no hem, turnover, stitching, joint, edging, tape of other material at the respective location that is not the single layer of the double-knit fabric. The leg openings may be seamless around at least one fourth of their periphery e.g. having only a seam in the crotch region along the longitudinal extension of the gusset. Alternatively, the leg openings may be seamless around a third, or two thirds of their periphery, such as around their complete periphery. The same may apply to the waist opening, which may also be seamless.

Different knitting techniques may be applied to provide the double-knit. The double-knit material may be an interlock knitted fabric. The body fabric may consist of a single layer of interlock knitted fabric throughout the garment.

The body fabric may be knitted with any suitable yarns such as those conventionally used for undergarments. Such yarns may include natural and/or synthetic fibres. Natural fibres may include cotton, silk, wool, cellulose, regenerated cellulose, rayon, viscose, modal, lyocell, Tencel, bamboo, hemp, flax, ramie, coir or banana. Suitable synthetic fibres may be selected from the group comprising polyamide, acrylic, polyester and elastane. The fibres may also be recycled fibres and can include both filament and staple fibres. The yarns may be relatively fine, since a double-knit fabric will inherently have greater weight than a single face counterpart. The yarns may be below 100 dtex, such as below 50 dtex or below 30 dtex.

The body fabric may be naturally stretchable by virtue of the knitting technique applied. Double-knit and interlock fabrics have good drape and stretch characteristics even when knitted with inelastic yarns so that the absorbent undergarment can provide a firm fit while at the same time adapting to the wearer's movements. At least some of the yarns comprising the knit may have elastic properties and can thus contribute to the overall elasticity of the garment, further improving fit and avoiding the need for a separate elastic at the waist and leg openings. Elastic fibres or yarns may include elastane in any appropriate form and amount and may be provided throughout the body fabric or only in certain parts of the body fabric. The elastic yarns and inelastic yarns may be knitted together from a single feed or from different feeds.

The undergarment may be of any suitable form and reference above and in the following to 'garment' is not intended to mean other than an undergarment i.e. an innermost garment intended for being in contact with the body of a user. The garment has a waist opening and two leg openings. It is however not excluded that closure elements e.g. between the waist opening and the leg openings may be provided, allowing easy removal or donning of the garment. The undergarment may have various designs, such as briefs, boxer, hipster, high waist, string, Brazilian, or other suitable and conventional undergarment designs.

The body fabric may be knitted as a single piece of material forming the front side, back side and crotch region. The single piece of material may be joined to itself at the waist by a conventional sewn seam. In the above discussion of seamless leg and waist openings, the presence of a joining seam extending perpendicular to the opening may still be considered 'seamless' in that the seam is not extending in a direction around the edge of the opening. Alternatively, the garment may be manufactured by circular knitting the body fabric and e.g. joining the front and rear sides at the crotch. Alternatively, the garment may be formed of a number of panels, joined to one another at appropriate locations. The skilled person will be aware of the alternative distributions of such panels and their advantages.

A central longitudinal axis of the undergarment may be defined from the rear side and towards the front side and a transversal crotch axis of the undergarment may be defined in a direction extending between the leg openings. The transversal crotch axis may be located at the narrowest point of the garment and/or may be located at the mid-point of the central longitudinal axis. The transversal crotch axis divides the crotch region into a front gusset region extending longitudinally between the transversal crotch axis and a front edge of the gusset, and a rear gusset region extending longitudinally between the transversal crotch axis and a rear edge of the gusset. The transversal crotch axis is perpendicular to the central longitudinal axis. For the purpose of the present disclosure, the crotch region is thus defined by the forward and rearward extent of the gusset.

There is also disclosed a method of manufacturing a washable absorbent undergarment, comprising: providing a web of double-knit material; cutting one or more panels from the web; joining the one or more panels to itself or each other to form the body fabric of the undergarment having a waist opening and leg openings; and attaching an absorbent gusset at an inside of a crotch region of the undergarment. By making use of double-knit material, such as the above-mentioned interlock knit fabric, the undergarment can be produced in simplified fashion, since cutting out of the pattern can take place without a requirement to hem or terminate the edges of the panels to prevent fraying.

In this manner, the undergarment can be completed without additional stitching or bonding extending around the leg openings. Furthermore, the undergarment may be completed without additional stitching or bonding extending around the waist opening.

In one configuration, the undergarment may comprise a single panel. In this case, the method may comprise joining the panel to itself at the hips to form both the leg openings and the waist opening.

The steps of the method may be carried out in any appropriate sequence. In particular, the gusset may be attached to the body fabric prior to, during or after, joining of the panel or panels. Attaching the gusset may comprise adhering a moisture barrier layer of the gusset to the body fabric.

### Brief description of the drawings

A washable absorbent undergarment according to the present disclosure will be described by way of example, with reference to the attached drawings, in which:
Fig. 1 shows a front view of an exemplary absorbent undergarment comprising an absorbent gusset;
Fig. 1A is a detail of Fig. 1;
Fig. 2 shows a top view of the undergarment of Fig. 1 in a flat laid-out state with the joints between the rear side and the front side removed;
Fig. 3 is a cross-sectional view through the undergarment of Fig. 2 in the direction III-III;
Fig. 4 is cut-away view of a portion of a of an absorbent undergarment;
Fig. 5 is a cross-sectional view through the undergarment of Fig. 4 taken in the direction V-V; and
Fig. 6 is a cross-sectional view through the undergarment of Fig. 4 taken in the direction VI-VI.

### Description

Different aspects of the present disclosure will be described more fully hereinafter with reference to the enclosed drawings. The reusable absorbent undergarment disclosed herein can, however be realized in many different forms, such as different sizes and absorption levels, and should not be construed as being limited to the aspects set forth herein. Reusable absorbent undergarments provide a more sustainable alternative to the commonly used disposable hygiene articles that are not intended to be re-used.

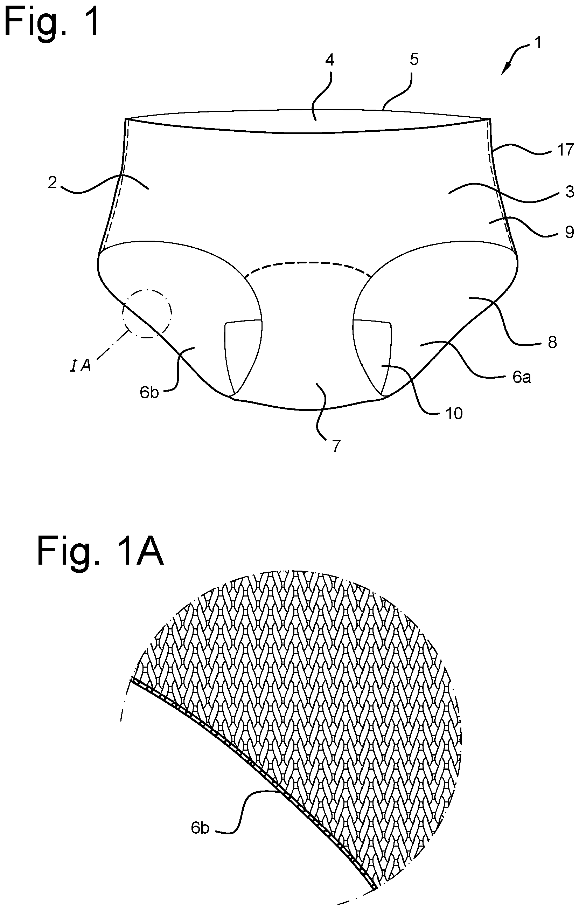

Fig. 1 illustrates an example of a washable and reusable absorbent undergarment 1 in the form of a slip. As outlined in the above, the undergarment 1 as proposed herein may however have various designs, such as briefs, boxer, hipster, high waist, string, Brazilian, or other suitable undergarment designs. The undergarment 1 may be designed for teens, male or female use.

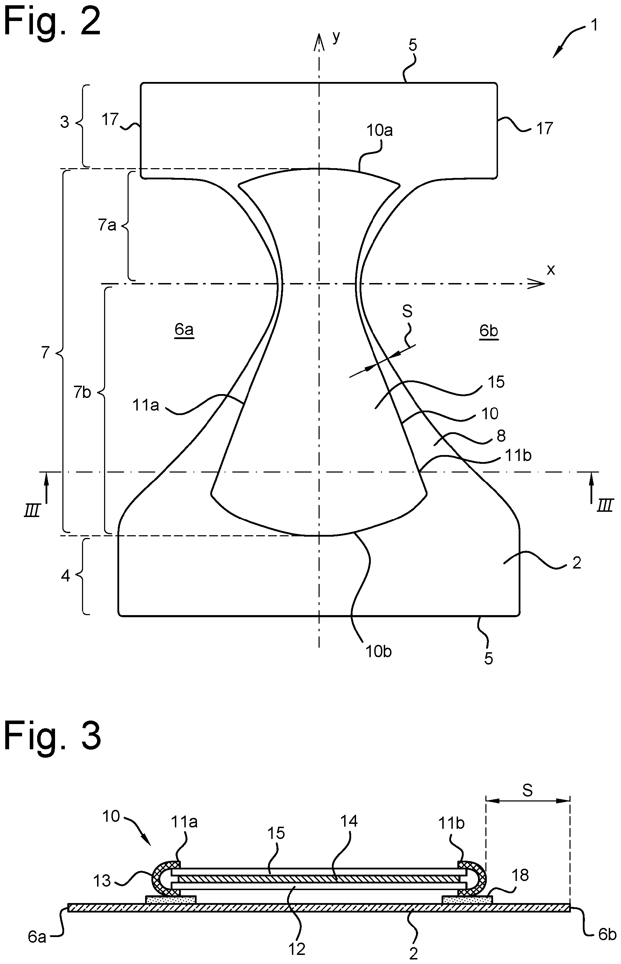

The undergarment 1 comprises a body fabric provided as one or more fabric panels 2 forming a front side 3, a rear side 4 and a crotch region 7 extending between the front and rear sides 3, 4. The front side 3 and the rear side 4 are joined at the hips such that the undergarment 1 forms a waist opening 5 and a pair of leg openings 6a, 6b. As such, the front side 3 will be visible from the front of the user when the undergarment 1 is worn, and the rear side 4 will be visible from the back of the user when the undergarment 1 is worn. The undergarment of the Fig. 1 and Fig. 2 example comprises one fabric panel 2, which is cut to form the front side 3, the rear side 4 and the crotch region 7. However, in other variants, a plurality of fabric panels may be joined together e.g. as a front panel, a back panel and a crotch panel. The one or more fabric panels may all comprise the same fabric, giving the undergarment a unitary appearance, or the one or more fabric panels may comprise different fabrics. In addition to the fabric panels, the undergarment may comprise additional body elements for example to provide aesthetically pleasing undergarments comprising e.g. lace fabric. In general however, for the presently intended purpose, the garment may be of the simplest possible design.

The body fabric is a single layer of interlock knitted fabric that terminates at the waist opening 5, without any waist band or additional elastic. There is also no seam, elastic or stitching around the leg openings 6a, 6b as illustrated in Fig 1A. A characteristic of interlock knitted fabric is that it can be cut and the cut edge is stable against threading without requiring any form of seam.

The body fabric may comprise any suitable fibres, including naturally derived fibres and mixtures thereof selected from the group consisting of cotton, wool, silk, cellulose, regenerated cellulose, rayon, viscose, modal, lyocell, Tencel, bamboo, hemp, flax, ramie, coir or banana. Alternatively, the fabric may be constructed of synthetic fibres or mixtures thereof selected from the group consisting of polyamide, acrylic, polyester. Further, the fabric may be constructed of a blend or a mixture of naturally derived and/or synthetic fibres. The fibres may be recycled fibres. The fabric may comprise a stretchable fabric, e.g. elastane, so that the absorbent undergarment can provide a firm fit while at the same time adapting to the wearer's movements thus preventing any leakage from migrating through the leg openings and keeping the absorbent gusset in place. The fabric may be breathable to allow vapor to escape from the wearer's skin and from the absorbent structure.

To join the front side 3 and the rear side 4 so as to form the waist opening 5 and the leg openings 6a, 6b, a pair of side joints 17 may be provided, as in the illustrated example. The side joints 17 and any other joints joining the one or more fabric panels 2 may be conventional joints in the art, such as seams made by conventional adhesive and/or conventional mechanical bonds, such as stitching techniques. As illustrated in Fig. 1, the fabric panel 2 has an outside surface 9 facing away from the wearer and an inside surface 8 facing in a direction towards the wearer. An absorbent gusset 10 is located in the crotch region 7 at the inside surface 8.

In Fig. 2, the absorbent undergarment 1 of Fig. 1 is shown in a flat laid-out state as seen from the inside surface 8, separated at the side joints 17. A central longitudinal axis y of the undergarment 1 is defined along the fabric panel 2 from the rear side 4 and towards the front side 3. Further, a transversal crotch axis x of the undergarment 1 is defined in a direction extending between the leg openings 6a, 6b, the transversal crotch axis x being perpendicular to the central longitudinal axis y at the crotch, being the position of minimum distance between the leg openings 6a, 6b.

The gusset 10 comprises a wearer facing top layer 15 that visibly illustrates to a user the extent of the gusset 10. The transversal crotch axis x divides the crotch region 7 into a front gusset region 7a extending longitudinally forwards from the transversal crotch axis x to a front edge 10a of the gusset 10, and a rear gusset region 7b extending longitudinally between the transversal crotch axis x and a rear edge 10b of the gusset 10. The longitudinal extension of the gusset 10 may depend for example on the design of the undergarment 1.

The gusset 10 thus defines the crotch region 7, being generally at a location where the need for absorbance is most prevalent. For undergarments intended primarily for night-time use, the gusset 10 may extend further rearwards and more of the gusset 10 may be located to the rear of the transversal crotch axis x extending further upwards towards the waist opening 5.

As exemplified by the illustrated undergarment 1, the gusset 10 also forms a pair of side edges 11a, 11b. Each side edge 11a, 11b is at least partly directed towards a respective leg opening 6a, 6b. Each side edge 11a, 11b, thus connects the front edge 10a and the rear edge 10b.

Close to the transversal crotch axis x and in the front gusset region 7a, each side edge 11a, 11b is arranged to follow the contour of the respective leg opening 6a, 6b of the undergarment 1 with a spacing of about 2 mm. In the rear gusset region 7b, the spacing between the gusset 10 and the leg openings 6a, 6b tapers to a maximum spacing of around 25 mm at the rear side.

The skilled person will recognise that the shape of the gusset 10 may alternatively be adapted to various absorption needs and to various designs of the undergarment 1, so as to provide sufficient absorption and satisfactory fit. For example, the side edges 11a, 11b may be straight or may comprise concave portions, as seen towards the central longitudinal axis y. Thus, the gusset 10 may be better adapted to fit between the wearer's legs. The front edge 10a of the gusset 10 may be curved convex as shown. This may be beneficial for the fit of the undergarment to the body and contribute to the avoidance of unwanted creases in the fabric panels. The front edge 10a may also be straight in order to reduce production and material costs. The same may apply to the rear edge 10b.

Fig. 3 shows a cross-section through the rear gusset region 7b, taken in the direction III-III in Figure 2. The body panel 2 can be seen to comprise a single layer of interlock knitted fabric without any seam or other reinforcement at the leg openings 6a, 6b. Beneath the top layer 15 is an absorbent core layer 14 and a moisture barrier layer 12. The top layer 15 is co-extensive with the barrier layer 12, while the core layer 14 is slightly smaller in area. The top layer 15 and barrier layer 12 are joined together around the side edges 11a, 11b and at the front and rear edges 10a, 10b by single sided adhesive tape 13. It will be understood that other methods of joining the layers of the gusset 10 may be employed.

The gusset 10 in Fig. 3 is permanently connected to the body panel 2 at the side edges 11a, 11b and also around the front and rear edges 10a, 10b by glue 18, at a spacing S from the leg openings 6a, 6b. The skilled person will understand that alternative or additional connections may be provided e.g. using double sided adhesive tape. Stitching may be provided along critical edges or at specific points where additional reinforcement is needed. The illustrated gusset 10 comprises just a top layer 15, absorbent core layer 14 and moisture barrier layer 12 but it will be understood that any number of additional layers may be provided depending upon requirements. Thus, further core layers may be provided with different sizes and absorbent characteristics. Wicking layers and distribution layers may also be provided. Any layers between the top layer 15 and the barrier layer 12 may be termed intermediate layers and together form the core layer 14. Although in the variant of Fig. 3, one intermediate layer is shown, it will be understood that different numbers of layers may be provided as discussed elsewhere.

The top layer 15 comprises a water-permeable material thus allowing the body fluids to migrate to the underlying core layer 14. The top layer 15 may be constructed of any suitable fabric, including naturally derived fibres selected from the group consisting of cotton, wool, silk, cellulose, regenerated cellulose, rayon, viscose, modal, lyocell, Tencel, bamboo, hemp, flax, ramie, coir or banana. Alternatively, the top layer may be constructed of synthetic fibres selected from the group consisting of polyamide, acrylic, polyester or elastane, such as a mixture of polyester and elastane. Further, the top layer 15 may be constructed of a blend or a mixture of naturally derived and/or synthetic fibres. The materials used for construction of the top layer should be soft and non-irritating to the skin and be readily penetrated by any body fluids. The top layer is washable and may be of a textile material, such as a knitted material. The top layer 15 may have a basis weight of 80-200 gsm.

The absorbent core layer 14 comprise any material capable of absorbing fluid, such as woven or nonwoven microfibre or polymer knits, fabric formed from hydrophilic fibres, absorbent fibres or powders. The absorbent core layer 14 may be of natural or synthetic fibres as described above for the top layer 15 but may alternatively be of polyester and polyamide.

The absorbent core layer 14 may also comprise a material having an open cell porous structures such as high loft or synthetic fibres having reservoir properties. The core layer may comprise between 20% and 100% naturally derived fibres, more preferably between 40% and 100% naturally derived fibres and most preferably between 60% and 100% naturally derived fibres. The basis weight of the core layer 14 may be 150-450 gsm.

The moisture barrier layer 12 may comprise a film or laminate e.g. of extruded polymeric material or a woven or knitted material of any suitable construction to prevent liquid from migrating from the gusset 10 to the fabric panel 2. The moisture barrier layer 12 in the illustrated variant, is a tightly woven activated carbon cloth that is sufficiently impermeable by nature. One suitable material is Flexzorb ^{®} from Chemviron SA. Laminates with polymer films may also be used. The activated carbon material may be highly desirable in reducing odours. The moisture barrier layer 12 may also comprise a coating of a moisture-impermeable material. The coating may be a polymer such as urethane wax or polyurethane provided for example on the surface facing away from the wearer of the moisture barrier layer 12.

The moisture barrier layer 12 is also preferably breathable, to allow vapour to escape from the absorbent undergarment 1 while preventing liquid from passing through the fabric layer. Air and vapour permeability may be achieved by the woven or knitted material or by an additional rate controlling layer attached thereto.

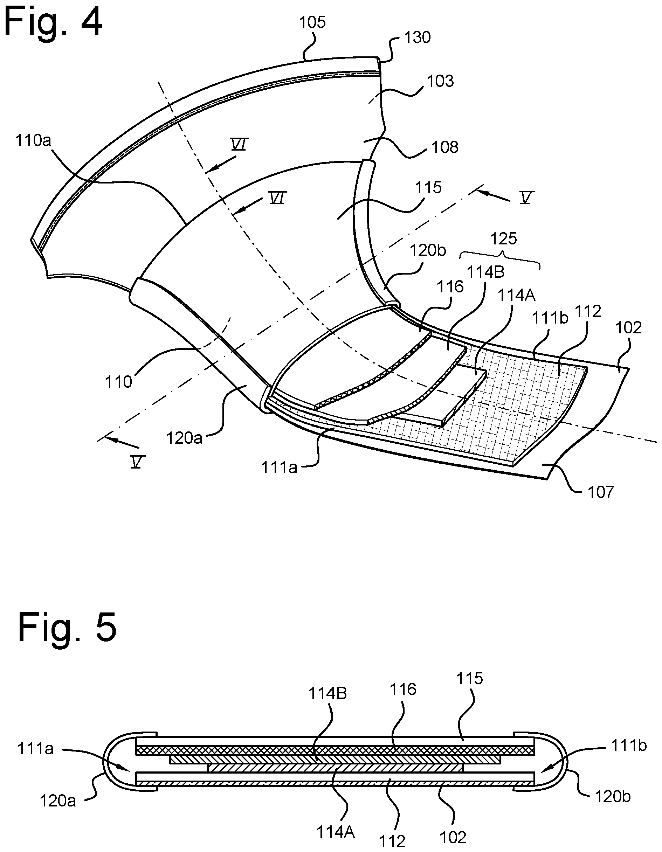

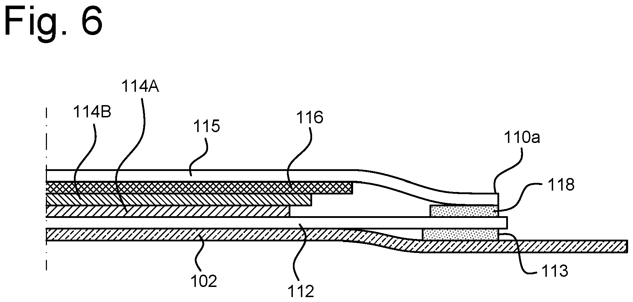

Fig. 4 shows part of a different garment 101 in which like references are preceded by 100. The garment 101 is viewed towards an inside surface 108 of the front side 103. Fig. 5 shows a cross-sectional view in the direction V-V of Fig. 4 and Fig. 6 is a cross-sectional view in the direction VI-VI.

A number of differences can be perceived in the garment 101 of Fig. 4 that distinguish over the garment 1 of Fig. 2. At the waist opening 105, an elasticated seam 130 is provided to ensure better hold-up.

Furthermore, the absorbent gusset 110 is provided with additional layers. In addition to a top layer 115, a barrier layer 112 and an absorbent core 114, there is provided a wicking layer 116. The wicking layer 116 is provided directly underneath the top layer 115 and may have wicking features to allow the moisture to spread away from the wearer and into the absorbent core 114. Wicking enables an efficient spread of the moisture, allowing the moisture to be received into a wider area of the underlying core layer 114. This feature is particularly relevant for users suffering from stress incontinence as spurts of urine may cause the core layer 114 to be saturated rapidly at the point of impact. By spreading the volume of the fluid over a wider receiving area, the wicking features of a wicking layer increase the overall absorptive capacity of the gusset 110. The wicking layer 116 may be constructed of any suitable fabric, including naturally derived fibres or mixtures thereof selected from the group consisting of cotton, wool, silk, cellulose, regenerated cellulose, rayon, viscose, modal, lyocell, Tencel, bamboo, hemp, flax, ramie, coir or banana. Alternatively, the wicking layer 116 may be constructed of synthetic fibres or mixtures thereof selected from the group consisting of polyamide, acrylic, polyester or elastane. Further, the wicking layer 116 may be constructed of a blend or a mixture of naturally derived and/or synthetic fibres. According to the present disclosure, the wicking layer 116 should be launderable. The wicking layer 116 may comprise between 20% and 100% naturally derived fibres, more preferably between 40% and 100% naturally derived fibres and most preferably between 60% and 100% naturally derived fibres. The basis weight of the wicking layer may be 180-250 gsm.

The gusset 110 of the illustrated absorbent undergarment 101 also contains two absorbent core sub-layers 114A, 114B, each capable of absorbing liquid and releasing the liquid during laundering. Release of the absorbed liquid is beneficial as it enables the absorbent undergarment 101 to be restored for reuse. The first core layer 114A, second core layer 114B and wicking layer 116 may be termed intermediate layers and together form a core 125. Although in Fig. 4, three intermediate layers are shown, it will be understood that different numbers of layers may be provided as discussed elsewhere.

The second core layer 114B is substantially coextensive with the wicking layer 116. The wicking layer 116 is slightly smaller in area than the top layer 115. The first core layer 114A is narrower than the second core layer 114B, ensuring additional absorptive capacity along the central longitudinal axis y of the garment 101, while reducing the thickness of the gusset 110 at its edges 111a, 111b.

Also shown in Fig. 4 and Fig 5, are bonding members 120a and 120b, whereby the gusset 110 is joined to the crotch region 107 using elastic bonding film that wraps around the first and second side edges 111a, 111b. It will be understood that other options for forming the bonding members may also be used. The body fabric 102 may, instead or in addition to bonding members 120a and 120b, extend and wrap around the first and second side edges 111a, 111b and bond to the top layer 115. The garment 101 thus has a connection along the edges of the gusset 110 that forms an effective seam for at least a part of the leg openings 106a, 106b in the crotch region 107, such as a fourth of the periphery of the leg opening. The leg openings 106a, 106b may be seamless around the remainder of their periphery.

Fig. 6 shows a shows a cross-sectional view in the direction VI-VI of Fig. 4, illustrating how the front edge 110a of the gusset 110 is attached to the fabric panel 102. The bonding members 120a and 120b of Fig. 5 extend only along the first and second side edges 111a, 111b and terminate at the front edge 110a and also at the rear edge 110b. In the illustrated embodiment, the front and rear edges of the gusset 110 are connected together by adhesive 118 joining the top layer 115 to the barrier layer 112. Additionally, the barrier layer 112 is adhered to the fabric panel by adhesive tape 113. It will be understood that other forms of connection may be employed and that additional stitching may be applied if local reinforcement is needed.

The disclosure may be varied within the scope of the appended claims. For example, the materials and dimensions used for the different layers forming the absorbent insert may be varied, as indicated above.

## Claims

1. A washable absorbent undergarment (1), comprising a body fabric (2) forming a front side, a rear side and a crotch region and defining a waist opening and leg openings, an absorbent gusset (10) at an inside of the garment comprising a garment-facing moisture barrier layer (12), wherein the body fabric comprises a single layer of double-knit material extending to the leg openings.

2. The garment of claim 1, wherein the gusset is spaced from the leg openings by at least 2 mm, preferably at least 5 mm.

3. The garment according to any one of the preceding claims, wherein the gusset is rectangular.

4. The garment according to any one of the preceding claims, wherein a spacing between the gusset and the leg openings tapers from a minimum distance in the crotch region to a maximum spacing.

5. The garment according to any one of the preceding claims, wherein the gusset comprises a body-facing top layer and optional intermediate acquisition and/or absorbent layer(s) between the top layer and the moisture barrier layer.

6. The garment according to any one of the preceding claims, wherein the body fabric extends and wraps around first and second side edges of the gusset in the crotch region.

7. The garment according to any one of the preceding claims, wherein the leg openings are seamless around at least one fourth of their periphery.

8. The garment according to any one of the preceding claims 1-5, wherein the leg openings are seamless around their complete periphery.

9. The garment according to any one of the preceding claims, wherein the waist opening is seamless.

10. The garment according to any one of the preceding claims, wherein the double-knit material is an interlock knitted fabric.

11. The garment according to any one of the preceding claims, wherein the gusset is permanently attached to the body fabric.

12. The garment according to any one of the preceding claims, wherein the body fabric consists of a single layer throughout the garment.

13. A method of manufacturing a washable absorbent undergarment, comprising:
- providing a web of double-knit material;
- cutting one or more panels from the web;
- joining the one or more panels to itself or each other to form the body fabric of the undergarment having a waist opening and leg openings, the fabric comprising a single layer of double-knit material extending to the leg openings; and
- attaching an absorbent gusset at an inside of a crotch region of the undergarment.

14. The method of claim 13, wherein the undergarment is completed without additional folding, stitching or bonding extending around at least one fourth of the periphery of the leg openings.

15. The method of claim 13, wherein the undergarment is completed without additional folds, bonds or stitches extending around the complete periphery of the leg and the waist openings.
